# EUROPEAN PATENT APPLICATION

(11) **EP 3 733 165 A1**
(43) Date of publication of application: **04.11.2020**
(21) Application number: 18896118.9
(22) Date of filing: 24.12.2018
(51) Int. Cl.: A61K 9/20, A61J 3/10, A61M 11/06, A61M 15/00

(54) **METHOD OF GENERATING MEDICATION IN AEROSOL FORM**

(30) Priority: 26.12.2017 EA 201800051
(71) Applicant: Limited Liability Company "Scientific-Industrial Innovation Deployment Center", Moscow, 121205 (RU)
(72) Inventor: BAKLANOV, Anatoly Maximovich, Novosibirskaya obl. 630051 (RU); VALIULIN, Sergey Vladimirovich, Novosibirsk 630064 (RU); ONISCHYK, Andrey Alexandrovich, Novosibirsk 630055 (RU); ABDIEV, Oleg Radzhabovich, Novosibirsk 630001 (RU)
(74) Representative: Osmans, Voldemars
(86) International application number: PCT/EA2018/000008
(87) International publication number: WO 2019/129339

(57) **Abstract**

Invention relates to the methods of obtaining of a medical aerosol. Object - development of a higher efficient method of drug generation into an aerosol solved via generation of a drug into an aerosol comprising supplying of heated air to the initial drug substance presented in a form of a multilayer tablet and evaporation of the initial drug substance followed by nucleation of the obtained supersaturated vapor and condensational growth of the formed aerosol particles and delivery of the obtained aerosol to patient's lungs. According to the method, the initial drug substance is prepared in the form of a multilayer, for example, a three-layer tablet, the upper and lower layers of which are formed of a drug and the middle layer is compressed cellulose, and during the aerosol generation the adhesive properties of which allow retaining the drug which was originally not only in the solid state, but also in the liquid state, or transitioned from solid to liquid state as a result of heating. The positive effect of the proposed method is achieved as a result of using the initial drug substance in a form of a multilayer tablet, which allows to efficiently evaporate and condensate a wide range of drugs that are both in solid and liquid state, including those that experience melting during heating. Advantages: precise dosing of the amount of drug transferred to aerosol; allow increase the efficiency of the conversion of drugs initial substance into an aerosol.

## Description

### Technical Field

The invention relates to methods of obtaining medical aerosols and can be applied in medicine for treatment of respiratory conditions as well as systemic diseases.

### Background Art

The aerosol drug delivery methods are increasingly widely used in the modern treatment methods of various diseases for drug delivery to the patient's lungs. This method of drug delivery is used to treat respiratory diseases such as asthma, bronchitis, cystic fibrosis, bacterial or fungal infection, chronic obstructive pulmonary disease, and primary pulmonary hypertension. A prior art discloses such drug delivery methods [1-3], moreover inhaled drug administration has a high potential for treatment of systemic diseases [4-7]. The major advantage of inhaled drug delivery is the fast therapeutic effect, simple administration, absence of necessity for healthcare stuff, absence of pain. Furthermore, the lungs have high solute permeability, a large surface area for absorption, limited proteolytic activity and pulmonary delivery is non-invasive. In addition, aerosol delivery has no restrictions associated with the use of water-insoluble drugs, unlike injection therapy.

Successful inhalation therapy depends on the precise delivery of the drug to the target area of the airway. The desirable area for delivering systemic drugs through the respiratory system is the alveolar area of the lungs, due to the wide surface area, ample blood supply and high permeability of the walls of the alveoli, which enables direct entry of drugs into the pulmonary and then into the systemic circulation. Aerosol administration methods will not only accelerate the therapeutic effect of the drug compared to oral administration, but will also reduce the administered dose, which can help reduce undesirable side effects of the drugs.

The efficiency of delivery of the aerosol particles to the alveolar lung area is defined by the size of the particles. As it is known based on published materials, the maximum deposition efficiency is shown by the particles of 10-20 nm diameter [5, 8 - 14]. The volume of the particles of such size that settle in alveolar area is 50%. Particles within the size range of 200 - 500 nm have reduced efficiency in depositing in alveolar area that reaches less then 10%, however the particles of 1- 3 micron demonstrate increased efficiency of alveolar deposition and reach - 20%. There are three types of inhalers used in modern practice of aerosol therapy: nebulizers, metered-dose aerosol inhalers and powder inhalers. All these inhalers allow generation of aerosol of particle size not lower then 1-3 micron. It is an obvious point that in order to ensure an efficient aerosol therapy the alternative methods will be a substantial addition that will allow high stable concentration of the particles in a wider size range including nanosize scale. One of the alternative methods is the thermal condensation method, based on the evaporation of the initial pharmaceutical substance of the drug followed by nucleation of supersaturated vapor and further the condensation growth of the formed aerosol particles. The thermal condensation method allows obtaining an aerosol with the particle size range from 3 nm to 10 micron with concentration up to 10⁸/cm⁻³. Such a high concentration allows delivery of a sufficient dose to the patient within a minimum time interval to achieve the desired therapeutic effect.

One of the problems related to the development of the aerosol drug administration method is the possibility of precise dose calculation. Therefore, it is necessary to develop such methods of aerosol drug administration that enable increase of the efficiency of converting the starting substance into an aerosol and allow precise determination of the amount of the drug delivered to the patient.

To date, there are a large number of devices that allow drug evaporation and subsequent condensation to form an aerosol. There is a method of generating aerosol by drug evaporation and subsequent condensation in a tube furnace, followed by cooling the resulting vapor by diluting it with air or inert gases [15]. The disadvantage of this equipment is the difficulty of determining the mass of the evaporated and condensed drug.

There is a device for generating medical aerosol that applies a pressed foamed graphite impregnated with a liquid composition containing pharmaceutical substances [16] as a vapor generating element. The disadvantage of this device is the complexity of manufacturing of the vapor generating element, the variation of drug concentration in aerosol during evaporation and inability to use pharmaceutical substances in solid form.

Closest to the claimed invention is the method of aerosol generation via a fast heating of thin film of drug without excipients, coated onto a solid surface of a metal cylinder [17]. This method allows to evaporate and then condense the drug to form aerosol of a particle size from several nanometers to several microns within about 50-300 ms. The disadvantage of this method is the possibility of drug application only as thin solid form films.

### Disclosure of invention

The object of the present invention is the development of an efficient method of generation of drugs in aerosol form using evaporation of the initial drug substance applied on a solid surface of a carrier allowing evaporation with subsequent condensation of drugs not only in solid state but also transferred from solid to liquid as a result of heating.

Technical result of the invention: aerosol administration methods not only accelerate the therapeutic effect of the drug compared to oral administration forms, but also reduce the administered dose, which can help reduce undesirable side effects of the drugs.

The presented method of drug generation in a form of an aerosol comprises supplying heated air to the initial drug substance, sublimation of the initial drug substance followed by nucleation of the obtained supersaturated vapor and delivery to the patient's lungs in a form of an aerosol. According to the invention, the initial substance of the drug is placed on an inert carrier (for example cellulose) in the form of a two- or three-layer tablet, which is produced using a mold, wherein a measured amount of the drug and cellulose is placed in the matrix alternately and then is pressed at a pressure in the range from 0.5 to 20 atm, in order that the drug is located either on one side or on both sides of the cellulose.

Advantageous effect of the presented method is reached as result of the fact that the initial drug substance - a multilayer tablet allows sublimating various range of drugs in both solid state and those that experience melting during heating. A liquid phase is held on the porous surface of the pressed cellulose due to the adhesion force. Furthermore, application of the tablet allows:
- Precisely dose the amount of drug transferred into an aerosol;
- Increase the efficiency of the conversion of drugs of the original substance into aerosol.

### Brief description of drawings

Fig. 1 - an example of a three-layer tablet with a drug and compressed cellulose: 1 - a drug layer, 2 - compressed cellulose, that due to it's adhesive properties allows retaining the drug which was initially not only in the solid state, but also in the liquid state, or transitioned from solid to liquid state as a result of heating.
Fig.2 - a dependency diagram of the concentration of aerosol obtained by evaporation and condensation of the drug - isoniazid, on the heating temperature of the air flow in the generator;
Fig.3 - a dependency diagram of the average size of the aerosol of isoniazid on the heating temperature of the air flow in the generator.

### Mode for Carrying Out the Invention

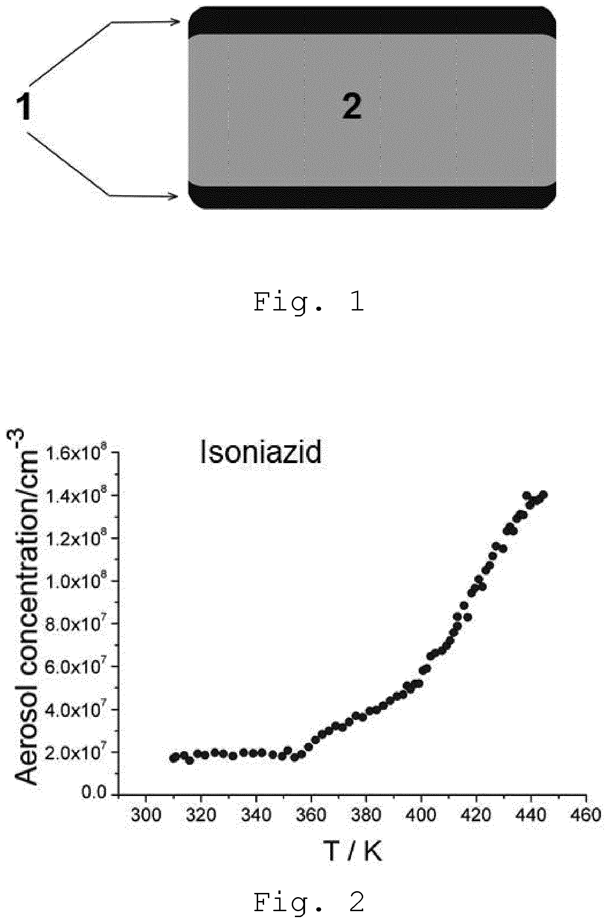

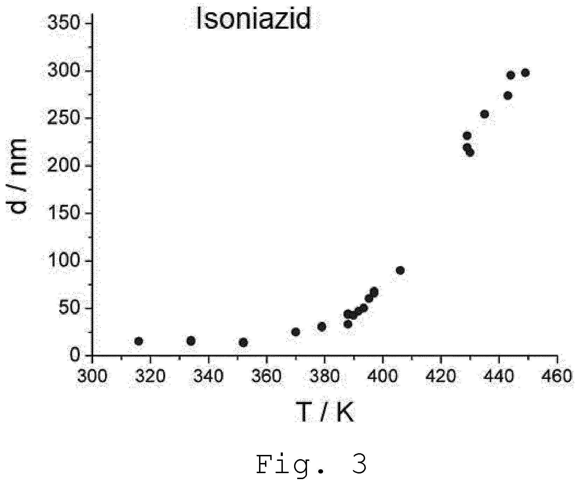

The method of generation of an aerosol of a pharmaceutical substance can be implemented as follows. Air is supplied at the inlet of an inhalation device for generating aerosol and heated to the required temperature indicated on the graphs (see Figs. 2 and 3).

Air heating temperature is within the range of 80 - 220 °C for a wide range of drugs. Further, the heated air is supplied to the drug substance in the form of a multilayer tablet with the drug 1 and a layer of pressed cellulose 2. A saturated vapor is formed in the result of evaporation of the drug substance from the surface of the tablet. After that the vapor is transferred with an air flow to the nucleation sector where it is cooling. As a result, the vapor becomes supersaturated, which leads to homogeneous nucleation, i.e. to aerosolization - the phase transition stage. The resulting aerosol of the drug is transferred with an air flow for therapeutic effect in the respiratory organs of the patient.

The tablet with a drug 1 and a cellulose layer 2 is an important element of the aerosol generation. The tablet is made as a "sandwich", the upper and lower layers of which are composed of a drug 1, and the inner layer is a pressed cellulose 2. The tablet is manufactured with a mold as follows: A layer of a drug, a layer of cellulose, a layer of a drug etc. are sequentially placed in the matrix alternately. Followed by pressing at a pressure in the range from 0.5 to 20 atm. The result is a stable three-layer tablet with a measured amount of a drug in it. A measured amount of a drug in the tablet allows precise dosing of the amount of the pharmaceutical substance in the generated aerosol and consequently the inhalation dose.

An example of implementation of the drug aerosolization.

The experiments were conducted aimed at confirmation of the implementation of the method with the drug - isoniazid. The tablet has been manufactured in which the lower drug layer was composed of 50 mg of powder isoniazid, middle layer - 100 mg of cellulose, the upper layer - 50 mg of powder isoniazid. The method of aerosol generation of the drug - isoniazid was performed on an experimental equipment. The results of the experimental works are presented in fig. 2, 3 - it demonstrates the dependency of the concentration and aerosol particle size of isoniazid obtained with the above-described method on the air heating temperature. Control of concentration and particle size or the drug aerosols was performed with an aerosol spectrometer [18].

### Reference sources:

1. Bailey, M.M., & Berkland, C.J. (2009). Nanoparticle Formulations in Pulmonary Drug Delivery. Medicinal Research Reviews, 29, 196 -212.
2. Gagnadoux, F., Pape, A.L., Lemarie, E., Lerondel, S., Valo, I., Leblond, V., Racineux, J.-L., & Urban, T. (2005). Aerosol delivery of chemotherapy in an orthotopic model of lung cancer. Eur. Respir. J. 26, 657-661.
3. Ruge, C.A., Kirch, J., & Lehr, C.-M. (2013). Pulmonary drug delivery: from generating aerosols to overcoming biological barriers - therapeutic possibilities and technological challenges. The Lancet Respiratory Medicine, 1, 402-413.
4. Agu, R.U., Ugwoke, M.I., Armand, M., Kinget, R., & Verbeke, N. (2001). The lung as a route for systemic delivery of therapeutic proteins and peptides. Respir. Res., 2, 198-209.
5. Labiris, N.R., & Dolovich, M.B. (2003). Pulmonary drug delivery. Part I: Physiological factors affecting therapeutic effectiveness of aerosolized medications. J. Clin. Pharmacol. 56, 588-599.
6. Laube, B.L. (2005). The Expanding Role of Aerosols in Systemic Drug Delivery, Gene Therapy, and Vaccination. Respiratory Care 50, 1161 - 1176.
7. Patton, J.S., Fishburn, C.S., & Weers, J.G. (2004). The Lungs as a Portal of Entry for Systemic Drug Delivery. Proc. Am. Thorac. Soc. 1, 338-344.
8. Edwards, D.A., Valente, A.X., Man, J., & Tsapis, N. (2003). Recent Advances Related to the Systemic Delivery of Therapeutic Molecules by Inhalation, in: Hickey, A.J. (Ed.) Pharmaceutical Inhalation Aerosol Technology, CRC Press, pp. 541 - 550.
9. Hinds, W.C., (1999). Aerosol Technology. Properties, Behavior, and Measurement of Airborn Particles. Second Edition, second ed. John Wiley & Sons, Inc., New York.
10. Heyder, J., (2004). Deposition of Inhaled Particles in the Human Respiratory Tract and Consequences for Regional Targeting in Respiratory Drug Delivery. Proc, Am, Thorac, Soc, 1, 315-320.
11. Hussain, M., Madl, P., & Khan, A. (2011). Lung deposition predictions of airborne particles and the emergence of contemporary diseases Part-I. theHealth 2, 51-59.
12. Oberdörster, G., Oberdörster, E., & Oberdörster, J. (2005). Nanotoxicology: an emerging discipline evolving from studies of ultrafine particles. Environmental Health Perspectives. 113: 823 - 839.
13. Wong, B.A. (2007). Inhalation Exposure Systems: Design, Methods and Operation. Toxicologic Pathology 35, 3-14.
14. Jaques, P.,A., & Kim, C.S. (2000). Measurement of total lung deposition of inhaled ultrafine particles in healthy men and women, Inhalation Toxicology. 12, 715-731.
15. Serebrennikov B.V.,Veliaminov A. and others Aerosol generator, utility model panent RU Nº 105564, MΠK A01G15/00, published 20.06.2011
16. Sternin J.I.,Okunevskiy M.B.,Moskalev E.V., Electronic inhaler, Inventor's certificate Nº 2014135145/12, MΠK A61M 11/00, published. 10.10.2015
17. Rabinowitz, J. D., M. Wensley, P. Lloyd, D. Myers, W. Shen, A. Lu, C. Hodges, R. Hale, D. Mufson, and A. Zaffaroni. (2004) Fast onset medications through thermally generated aerosols. The Journal Of Pharmacology And Experimental Therapeutics 309: 769-775.
18. Dubtsov, S., Ovchinnikova, T., Valiulin, S., Chen, X., Manninen, H.E., Aalto, P.P., Petäjä, T. (2017) Laboratory verification of Aerosol Diffusion Spectrometer and the application to ambient measurements of new particle formation, Journal of Aerosol Science, 105: 10 - 23.

## Claims

1. A method of generation of an aerosol of a drug comprising supplying heated air to an initial drug substance, evaporating the initial drug substance with a subsequent nucleation of the obtained supersaturated vapor and delivering the drug to patient's lungs in a form of the aerosol, **characterized in that** the initial drug substance in made in a form of a multi-layer tablet manufactured with a press mould wherein an inert carrier and a measured amount of the drug are alternately loaded in the matrix of the press mould and subsequently pressed at a pressure in a range from 0,5 to 20 atm.
